# EUROPÄISCHE PATENTANMELDUNG

(11) **EP 2 062 554 A1**
(43) Veröffentlichungstag der Anmeldung: **27.05.2009**
(21) Anmeldenummer: 09003004.0
(22) Anmeldetag: 23.08.2005
(51) Int. Cl.: A61F 2/44, A61F 2/30

(54) **Implantat für den chirugischen Einsatz bei Menschen oder Wirbeltieren**

(30) Priorität: 25.08.2004 DE 102004041354
(62) Teilanmeldung aus: 05018303.7
(71) Anmelder: Buck, Alfred Ernst, 71149 Bondorf (DE)
(72) Erfinder: Buck, Alfred Ernst, 71149 Bondorf (DE); Kaps, Hans-Peter, Dr., 72076 Tübingen (DE)
(74) Vertreter: Blumbach - Zinngrebe

(57) **Zusammenfassung**

Die Erfindung betrifft ein Verfahren zur Herstellung eines Implantats sowie ein Implantat für den chirurgischen Einsatz bei Menschen oder Wirbeltieren, insbesondere zum Ersatz, zum Teilersatz oder zur Verstärkung einer beschädigten Bandscheibe oder zum Ersatz, zum Teilersatz oder zur Verstärkung eines anatomischen Gelenks. Das Implantat umfaßt dabei zumindest einen Draht oder zumindest eine Faser. Der Draht oder die Faser liegen zumindest teilweise als Maschenware vor und sind dabei vorzugsweise Teil eines Gestricks.

## Beschreibung

Die Erfindung betrifft ein Implantat für den chirurgischen Einsatz bei Menschen oder Wirbeltieren, insbesondere zum Ersatz, zum Teilersatz oder zur Verstärkung einer beschädigten Bandscheibe oder zum Ersatz, zum Teilersatz oder zur Verstärkung eines anatomischen Gelenks.

Das Stützgerüst des menschlichen Körpers, das Skelett, ist aus Knochen und Knorpel zusammen gesetzt. Gemeinsam mit Sehnen, Bändern und Muskeln bildet es den Bewegungsapparat bei Menschen und Wirbeltieren.

Dabei dienen die Gelenke als Verbindungsstellen zwischen den Knochen und sind für die Beweglichkeit des Körpers zuständig. Neben dem Gewährleisten der Beweglichkeit leisten die Gelenke dabei vor allem noch das Auffangen bzw. Kompensieren des beim Stehen und Aufspringen entstehenden Drucks auf das Skelettsystem. Diese Aufgabe kommt auch der bei Mensch und Wirbeltieren zwischen je zwei benachbarten Wirbeln liegenden Bandscheibe (auch Zwischenwirbelscheibe genannt) zu.

Beispielsweise werden beim Menschen allein schon durch das aufrechte Stehen und Gehen die Wirbelgelenke und die Gelenke in Hüfte und Knie beansprucht. Durch Abnutzungsprozesse, unter anderem bedingt durch Alter, Krankheit oder Überbeanspruchung, entsteht ein Verschleiß der Gelenke oder Wirbelgelenke bzw. der Gelenkoberflächen. Dies führt unter Umständen zu Bewegungseinschränkungen, Kraftminderung oder Schmerzen. Dies kann aber durch einen Ersatz, einen Teilersatz bzw. eine Verstärkung der Gelenke mittels eines Implantats kompensiert werden.

Dabei sollte das Implantat im Wesentlichen den Abmessungen und der Form des zu ersetzenden Gelenks oder der zu ersetzenden Bandscheibe entsprechen, eine ausreichende Biokompatibilität mit dem umliegenden Gewebe und vor allem vergleichbare physikalische Eigenschaften, insbesondere bezüglich Steifigkeit, Elastizität, Federung und Dämpfung aufweisen und eine im Rahmen der natürlichen Bewegung uneingeschränkte Bewegung ermöglichen und eine lange Lebenszeit besitzen, um einen Patienten nicht durch häufige Implantatwechsel zu belasten.

Dazu hat es eine Vielzahl von verschiedenen Implantaten gegeben. Beispielsweise werden bei einem Gelenk, wie einem Schulter- Hüft-, oder Kniegelenk, Gelenkkugel bzw. Gelenkkopf und Gelenkpfanne mit Implantaten aus Kunststoff getauscht, welche fest im Knochen verankert werden müssen. Es wird in diesem Zusammenhang unterschieden zwischen der Totalendoprothese, bei der sowohl Gelenkkopf als auch Gelenkpfanne ersetzt werden, während bei der Teilendoprothese nur der Gelenkkopf ausgetauscht wird.

Weitere bekannte Werkstoffe bzw. Werkstoffkombinationen zum Bilden der Gelenkpaarung, d.h. zum Bilden von Gelenkpfanne und Gelenkkopf bzw. zum Bilden der aneinander reibenden Oberflächen von Gelenkpfanne und Gelenkkopf umfassen die Paarung von Metall-Metall, Kunststoff-Metall, Kunststoff-Keramik oder Keramik-Keramik. Bei der Auswahl der Werkstoffkombination spielen neben der Biokompatibilität auch die Langzeitverankerung des Implantats im Körper, der Verschleiß und der Abrieb an den Gelenkoberflächen des künstlichen Gelenks eine entscheidende Rolle. Dabei hängt der Abrieb von den Werkstoffen der Gelenkpaarung ab. Kunststoffe weisen nachteilig einen erhöhten Verschleiß aufgrund eines erhöhten Abriebs gegenüber zueinander präzise geschliffenen Metalloberflächen oder Keramikoberflächen auf.

Die Strukturen der Knochen bzw. der Gelenke sind in ihrer Funktion, vor allem bezüglich bezüglich Steifigkeit, Elastizität, Federung und Dämpfung, so angepaßt, daß sie der örtlichen Beanspruchung optimal gerecht werden. Diese funktionell angepaßte Struktur wird durch den Einbau eines Implantats aufgrund eines veränderten Dehnungsverhaltens und einer veränderten Kraftübertragung von dem Implantat, vor allen Dingen bei Metallen oder Keramiken, in den Knochen gestört. Die Kraftübertragung von dem Implantat in den Knochen spielt dabei eine entscheidende Rolle bei der Frage der Langzeitverankerung bzw. -befestigung des Implantats im Körper.

Um zudem ein festes Verwachsen des Implantats mit dem Knochen durch Knocheneinwuchs in das Implantat zu gewährleisten, werden die auf die zum Knochen hin orientierten Außenseiten der Implantate mit einer Oberflächenstrukturierung versehen.

Zum Ersatz von Bandscheiben offenbart beispielsweise die Schrift DE 696 23 535 T2 eine künstliche Bandscheibe, die ein zweiteiliges, hartes, chrombeschichtetes metallisches Kugelgelenklagersystem aufweist und eine uneingeschränkte Bewegung ermöglicht, dabei aber keine Fähigkeit besitzt, Stöße auf die Wirbelsäule zu absorbieren. Zudem müssen die sich an- bzw. gegeneinander bewegenden Oberflächen des Kugelgelenklagersystems präzise und daher aufwendig bearbeitet werden, um einen beschleunigten, unter anderem durch Reibung bedingten, Verschleiß der Oberflächen zu vermeiden bzw. zu minimieren.

Das Dokument DE 694 28 143 T2 offenbart zum Ersatz einer beschädigten Bandscheibe eine Bandscheibenprothese, die eine obere steife Platte, eine untere steife Platte und einen elastomeren Kern, der zwischen den Platten angeordnet ist und an diesen anliegt, umfaßt. Diese Implantat weist durch seinen elastomeren Kern eine Stoßabsorption und bei geringer Steifheit eine ausreichende Beweglichkeit auf. Jedoch kann der elastomere Kern mit der Zeit vom Körper abgebaut werden. Zudem neigen Elastomere dazu, sich mit der Zeit zu deformieren und dadurch ihre Abmessungen und Eigenschaften zu verändern.

Beide Implantate weisen auf den in Richtung der Wirbel orientierten Außenseiten Vorsprünge auf, um das Implantat in den Wirbeln zu fixieren. Zudem werden bei beiden Implantaten die genannten Außenseiten noch mit einem porösen Überzug versehen, um ein Einwachsen von Gewebe bzw. den Knocheneinwuchs in das Implantat durch die umliegenden Wirbel zu ermöglichen und dadurch den Knochen fester mit dem Implantat zu verbinden.

Vor diesem Hintergrund liegt daher der vorliegenden Erfindung die Aufgabe zugrunde, die oben genannten Nachteile des Standes des Technik, nicht nur für Implantate zum Ersatz, zum Teilersatz oder zur Verstärkung von Bandscheiben sondern auch für Gelenke, zu vermeiden.

Dies umfaßt dabei das Ziel, ein Implantat bereitzustellen, welches im Rahmen der natürlichen Bewegung (Biege-, Kipp- und Drehbewegungen oder aber auch Translationsbewegungen) eine uneingeschränkte Bewegung ermöglicht und zudem durch seine Elastizität, seine Federung und innere Dämpfung die Eigenschaft der Stoßabsorption aufweist.

Zudem soll das Implantat biokompatibel sein und dabei noch eine Oberfläche besitzen, welche ein Einwachsen von Gewebe ermöglicht, um eine feste Verbindung zwischen dem Implantat und dem umliegenden Gewebe und dadurch eine verbesserte Stabilität zu gewährleisten, ohne daß es erforderlich ist, zusätzliche Vorrichtungen an dem Implantat zum Fixieren in dem Gewebe anzubringen.

Darüber hinaus soll das Implantat möglichst formstabil und verschleißarm sein und vom Körper auch über einen längeren Zeitraum nicht unerwünscht abgebaut oder in seinen Abmessungen verändert werden können und keine bzw. zumindest nur geringe Abnutzungsschäden durch die Knochenoberfläche zugefügt bekommen.

Ferner soll das Implantat in einem einfachen Verfahren kostengünstig herzustellen und durch herkömmliche auf diesem Gebiet bekannte orthopädische Chirurgie einzusetzen sein.

Insbesondere soll das Implantat durch eine einfache Variation der Herstellungsparameter, insbesondere bezüglich seiner Steifigkeit, Elastizität und seiner Federung und inneren Dämpfung, an die gewünschten Eigenschaft bzw. Spezifikationen des entsprechend zu ersetzenden bzw. zu unterstützenden Gelenks oder der Bandscheibe angepaßt werden können, um eine bestmögliche Kraftübertragung von dem Implantat in den Knochen bzw. das umliegende Gewebe zu gewährleisten.

Da ein aus mehreren Bestandteilen aufgebautes Implantat die Herstellung als auch den chirurgischen Einbau in den Körper komplizieren und verteuern können, sollte das Implantat einen vereinfachten Aufbau aufweisen und unter Umständen sogar vorteilhaft nur einteilig ausgebildet sein.

Gelöst wird diese Aufgabe auf überraschend einfache Weise bereits durch das Implantat gemäß den Merkmalen des Anspruchs 1 und dem Verfahren zur dessen Herstellung gemäß Anspruch 25. Vorteilhafte Ausführungsformen sind Gegenstand der jeweiligen Unteransprüche.

In einer ersten Ausführungsform umfaßt die Erfindung ein Implantat für den chirurgischen Einsatz bei Menschen oder Wirbeltieren zum Ersatz, zum Teilersatz oder zum Verstärken einer beschädigten Bandscheibe oder zum Ersatz, zum Teilersatz oder zum Verstärken eines Gelenks, welches zumindest einen Draht und/oder zumindest eine Faser umfaßt.

Der Draht und/oder die Faser liegt bzw. liegen zumindest teilweise als Maschenware vor. Es ist dabei zu verstehen, daß zumindest ein oder mehrere Drähte bzw. Fasern pro Maschenware verwendet werden können. Maschenware weist von sich aus eine große Elastizität auf, die sich günstig auf die Schmiegsamkeit der daraus hergestellten Implantate auswirkt.

Bei einer besonders vorteilhaften Ausführungsform ist der Draht oder die Faser Teil eines Gestricks. Unter Gestrick versteht man nacheinander laufende Maschen, wobei jede neue Schlinge, aus der eine Masche gebildet wird, durch die Masche vorher gezogen wird. So bildet sich ein besonders poröses und dehnungsfähiges Material. Ein aus einem Drahtgestrick aufgebautes Implantat weist bei Biege- als auch bei Druckbeanspruchungen sowohl eine hohe Elastizität als auch eine hohe innere Dämpfung auf.
Dies begründet sich dadurch, daß eine einwirkende Kraft sich über und auf eine Vielzahl von Kontakten der einzelnen Drähte und/oder Fasern verteilt. Durch innere Reibung der Drähte und/oder Fasern in den einzelnen Maschen werden Schwingungen, wie sie von außen auf das Körpersystem, Bandscheibe und/oder Gelenke durch Gehen, Laufen und vor allem durch Springen eingeleitet werden, somit wirksam gedämpft. Das erfindungsgemäße Drahtgestrick ist dabei in seinem Hub zuverlässig weit deformierbar und nimmt unter Druck bis zu einer Minimaldicke ab.

Da das Implantat eine im Rahmen der natürlichen Bewegung (Biege-, Kipp- und Drehbewegungen oder aber auch Translationsbewegungen) uneingeschränkte Bewegung ermöglichen soll und zudem die Eigenschaft besitzen soll, Stöße abzufedern und die Stoßenergie zu absorbieren, ist es vorteilhaft, wenn der genannte Draht und/oder die genannte Faser elastisch bzw. biegelastisch, selbstverständlich biokompatibel und zusätzlich noch verschleißarm bzw. abriebfest ist und vom Körper auch über einen längeren Zeitraum nicht unerwünscht abgebaut werden kann.

Daher ist der Draht und/oder die Faser dadurch gekennzeichnet, der er bzw. sie als Bestandteil zumindest Titan umfaßt. Ein weiteres biokompatibles Material, welches in der vorliegenden Erfindung einsetzbar ist, ist unter anderem Edelstahl oder auch eine Edelstahllegierung. Weitere einsetzbare Materialien, die allesamt gegenwärtig in medizinischen Implantatanwendungen verwendet werden, sind Legierungen, die unter anderem als Bestandteile in verschiedenen Mengenanteilen Titan, Kobald, Chrom, Aluminium, Vanadium, Niob und/oder Zirkonium enthalten oder stabile Kunststoffe.

Wie sich im Laufe der Beschreibung noch ergibt, kann aber auch unter Umständen die Verwendung eines durch den Körper resorbierbaren Materials sinnvoll sein, d.h. welches vom Körper mit der Zeit abgebaut werden kann. Verwendbare Materialien sind insbesondere resorbierbare organische Materialien, dabei unter anderem Catgut, Catgut Chrom oder Kollagen oder aber auch resorbierbare synthetische Materialien, wie organisch abbaubare Polymere, dabei unter anderem Vicryl, Polysorb, Dexon, Piralac, Serafit, Bondek, Maxon oder Panacryl.

Zudem können eines oder mehrere der Materialien in einem Draht und/oder Faser integriert sein. Die genannten Materialien sind beispielhaft zu verstehen und beschränken sich keinesfalls auf die genannte Auswahl.

Bei der Wahl der Draht- und/oder Faserstärke ist zu beachten, daß der Durchmesser eines Drahtes und/oder einer Faser mit seiner Lebensdauer verknüpft ist. Um eine Bruchgefahr zu vermeiden, sollte der Durchmesser eines Drahtes und/oder einer Faser nicht zu dünn gewählt sein. Der Draht und/oder die Faser weisen dabei einen Durchmesser von etwa 0,01 mm bis 5 mm, bevorzugt von etwa 0,05 mm bis etwa 1 mm und besonders bevorzugt von etwa 0,2 mm bis etwa 0,3 mm aufweist bzw. aufweisen.

Neben der Wahl des Draht- bzw. Fasermaterials werden die Eigenschaften der Maschenware aber auch durch die Größe und Dichte der Maschen beeinflußt, wobei die Dichte der Maschen in einem formgebenden Verfahrensschritt erhöht werden kann. Dies hat Auswirkungen auf die Eigenschaften der Maschenware bzw. des Implantats, insbesondere auf die Elastizität und damit auch Federung und die Stoßabsorption des Implantats.

Die Maschenware weist dabei eine Maschenweite von 0.01 mm bis 50 mm, bevorzugt von 0.5 mm bis 20 mm und besonders bevorzugt von 3 mm bis 8 mm auf.

Neben dem Material, dem Durchmesser und der Maschenweite ist auch die Art der Herstellung der Maschen für die Funktionalität des Implantats von Bedeutung. Wie bereits erwähnt, wird vorteilhaft eine gestrickte Maschenware verwendet. Diese kann dabei auch mit mehreren Drähten und/oder Fasern, z.B. doppelfädig, ausgearbeitet sein. Prinzipiell kann die Maschenware aber auch als gehäkelte oder gewirkte Maschenware vorliegen.

Insbesondere hat sich aber herausgestellt, daß eine gestrickte und insbesondere eine rundgestrickte Maschenware zu bevorzugen ist. Vor allem hat es sich als besonders vorteilhaft bei der Herstellung erwiesen, wenn das Maschenmaterial rundgestrickt wird und die Maschenware als gestrickter Schlauch vorliegt.

Alternativ kann der Draht und/oder die Faser aber auch als Gelege, Geflecht und/oder als Gewebe vorliegen.

In einer weiteren Ausführungsform kann dabei die Dichte der Maschen erhöht werden und zwar, wenn die Maschenware als gepreßte Maschenware vorliegt. Dies geschieht in einem formgebenden Verfahrensschritt. Dies hat dabei Auswirkungen auf die Eigenschaften und verbessert insbesondere die Elastizität und damit die Feder- und Dämpfungseigenschaften des Implantats und somit die Fähigkeit, Stöße zu absorbieren.

Dabei hat es sich als vorteilhaft erwiesen, wenn die Maschenware als aufgerollte, gewickelte und/oder gefaltete Maschenware vorliegt und dann gepreßt wird. Durch das Pressen verhaken und/oder verklemmen sich der Draht und/oder die Faser der Maschenware untereinander und ineinander, wodurch diese miteinander verbunden sind.

Die Maschenware liegt nach dem Preßvorgang als gepreßter Formkörper vor und weist die Form eines gewünschten Formkörpers auf. Durch die Maschenware bilden sich dabei innerhalb des gepreßten Formkörpers Hohlräume aus.

Diese neuartigen Implantate zeigen je nach Verdichtung der Maschenware durch den Preßvorgang eine mehr oder wenige große Kompressibilität, was den Implantaten eine gewisse Eigenfederung und innere Dämpfung verleiht.

Die durch den Preßvorgang bestimmte Größe der Hohlräume bestimmen die Eigenschaften des genannten Formkörpers hinsichtlich Steifigkeit, Elastizität bzw. Biegeelastizität, Kompressibilität, Federung und Dämpfung.

Dabei werden die Elastizität und Dämpfung und alle anderen Parameter (z.B. Abmessungen) gemäß des natürlichen zu ersetzenden Implantats gewählt.

Der gebildete Formkörper besitzt die Fähigkeit, sich den Beanspruchungen durch elastische oder plastische Verformungen ohne bleibende Schäden durch seine Schmiegsamkeit anzupassen und ist unempfindlich gegen Verkantungen und Durchbiegungen und verleiht dem Menschen bzw. dem Wirbeltier die Fähigkeit, Biege-, Kipp- und Drehbewegungen oder aber auch Translationsbewegungen durchzuführen.

Bei einer besonders bevorzugten Ausführungsform der Erfindung weist der Formkörper eine Federkonstante auf, die bei elastischer Verformung ansteigt. Dies gewährleistet eine optimale Federung und Dämpfung des Formkörpers bzw. des Implantats. So federt das Implantat anfangs weich und wird mit zunehmender Verformung härter. Dies ist besonders für Bandscheibenprothesen geeignet.

Das Implantat weist dabei eine Federkonstante auf, die zwischen 5 und 3000 N/mm liegt. Insbesondere sind anfänglich Federkonstanten, also in nicht belastetem Zustand von 5 und 300 N/mm, bevorzugt zwischen 15 und 200 N/mm, besonders bevorzugt zwischen 30 und 130 N/mm vorgesehen. Mit zunehmender Verformung nimmt die Federkonstante zu. Insbesondere nimmt die Federkonstante mit zunehmender Verformung etwa linear bis quadratisch zu, so daß sich ein etwa quadratischer bzw. kubischer Kraft-Weg-Verlauf ergibt. Dabei kann das Implantat einen veränderten Kraft-Weg-Verlauf nach dem Einbau in den Körper durch das anliegende Gewebe bzw. den anliegenden Knochen aufzeigen.

Da der Formkörper durch die verdichtete Maschenware gebildet wird und somit neben den Hohlräumen eine rauhe Oberfläche aufweist, und somit poröse Eigenschaften aufweist, ermöglicht dies ein Einwachsen von Gewebe und dadurch den umgebenden Knochen fester mit dem Implantat zu verbinden. Zudem gestatten die Hohlräume die Aufnahme des beim Reiben des Implantats an dem umgebenden Gewebe bzw. Knochen auftretenden Gewebe- bzw. Knochenabrieb, so daß dieser Abrieb nicht zwischen den aneinandergleitenden Oberflächen verbleibt.

In den konkreten Ausführungsformen sollten die Implantate im wesentlichen anatomisch geformt sein, d.h. die Abmessungen und die Form des Implantats im wesentlichen den Abmessungen und der Form der zu ersetzenden Bandscheibe und/oder des zu ersetzenden anatomischen Gelenks entsprechen. Jedoch kann es aber auch aus Gründen der Funktionalität bei der Herstellung und beim Einbau des Implantats unter Umständen vorteilhaft sein, eine andere Form zu wählen.

In einer Ausführungsform ist der Formkörper nierenförmig mit einer gebogenen konvexen Seite und einer gegenüberliegenden konkaven Seite ausgebildet und entspricht dabei im wesentlichen der Form einer natürlichen Bandscheibe.

In einer weiteren Ausführungsform entspricht der gebildete Formkörper im wesentlichen der Form einer natürlichen Hüftgelenkpfanne oder eines natürlichen Hüftgelenkkopfs.

In einer weiteren Ausführungsform entspricht der gebildete Formkörper im wesentlichen der Form einer natürlichen der Schultergelenkpfanne oder eines natürlichen Schultergelenkkopf.

Die genannten Ausführungsformen sind beispielhaft zu verstehen und beschränken sich keinesfalls auf die genannte Auswahl. Ohne weitere Gelenke im Detail darzustellen, kann das erfindungsgemäße Implantat auch zum Ersatz, zum Teilersatz oder zum Verstärken für andere Gelenke, beispielsweise Kniegelenke, Sprunggelenke, Ellenbogengelenke, Fingergelenke oder Zehengelenke, beim Menschen oder bei Wirbeltieren chirurgisch eingesetzt werden.

Wie bereits beschrieben, kann unter Umständen die Verwendung eines durch den Körper resorbierbaren Materials sinnvoll sein. Dies ist in der besonders vorteilhaften Ausführungsform gegeben, bei der das Implantat mit Stammzellen bzw. mit körpereigenen Zellen, welche im Labor gezüchtet werden, besetzt wird. Der große Vorteil dabei ist, daß das neue Implantat sich fest mit dem Knochen verbindet, während beispielsweise Implantate aus Kunststoff oder anderen Materialien sich mit der Zeit lockern und/oder zu Fremdkörperreaktionen führen können. Dabei dient die das Implantat formende Maschenware als Stützgerüst. Die Maschenware kann eines oder mehrere der genannten resorbierbaren Materialien umfassen und/oder eines oder mehrere der genannten biokompatiblen Materialien, insbesondere Titan, welches dann dauerhaft im Körper als Stützgerüst verbleibt.
Der Drahtdurchmesser und/oder die Maschenweite wird bzw. werden dabei so gewählt, um ein einfaches Einwachsen der Stammzellen bzw. der körpereigenen Zellen zu ermöglichen.

Wird beispielsweise mit Stammzellen besetztes Catgut verwendet, ist sogar denkbar, daß das Implantat sich zumindest teilweise zu natürlichem Gewebe umbildet.

Im weiteren umfaßt die vorliegende Erfindung ein Verfahren zur Herstellung eines Implantats für den chirurgischen Einsatz bei Menschen oder Wirbeltieren, insbesondere zur Herstellung eines Implantats gemäß der vorangehenden Beschreibung, zum Ersatz, zum Teilersatz oder zum Verstärken einer beschädigten Bandscheibe oder zum Ersatz, zum Teilersatz oder zum Verstärken eines anatomischen Gelenks, welches das Bereitstellen zumindest eines Drahtes und/oder zumindest einer Faser umfaßt.

Dabei wird zumindest ein Draht und/oder zumindest eine Faser bereitgestellt, der bzw. die als Bestandteil zumindest Titan aufweist bzw. aufweisen.

Alternativ dazu kann auch zumindest ein Draht und/oder zumindest eine Faser bereitgestellt werden, der bzw. die als Bestandteil zumindest ein biokompatibles Material, insbesondere Edelstahl oder eine Edelstahllegierung aufweist bzw. aufweisen. Weitere einsetzbare Materialien, die allesamt gegenwärtig in medizinischen Implantatanwendungen verwendet werden, sind Legierungen, die unter anderem als Bestandteile in verschiedenen Mengenanteilen Titan, Kobald, Chrom, Aluminium, Vanadium, Niob und/oder Zirkonium enthalten oder stabile Kunststoffe.

Eine weitere Möglichkeit besteht in dem Bereitstellen zumindest eines Drahtes und/oder zumindest einer Faser, welcher bzw. welche als Bestandteil zumindest ein durch den Körper resorbierbares Material aufweist bzw. aufweisen. Verwendbare Materialien sind insbesondere resorbierbare organische Materialien, dabei unter anderem Catgut, Catgut Chrom oder Kollagen oder aber auch resorbierbare synthetische Materialien, wie organisch abbaubare Polymere, dabei unter anderem Vicryl, Polysorb, Dexon, Piralac, Serafit, Bondek, Maxon oder Panacryl.
Der Draht und/oder die Faser wird bzw. werden mit einem Durchmesser von etwa 0,01 mm bis 5 mm, bevorzugt von etwa 0,05 mm bis etwa 1 mm und besonders bevorzugt von etwa 0,2 mm bis etwa 0,3 mm bereitgestellt.

Es hat sich dabei als besonders vorteilhaft erwiesen, wenn der Draht und/oder die Fasern zumindest teilweise als Maschenware bereitgestellt wird bzw. werden. Dabei kann auch ein oder mehrere Drähte/Fasern pro Maschenware verarbeitet sein.

Die Maschenware wird dabei mit einer Maschenweite von 0.01 mm bis 5 mm, bevorzugt von 0.1 mm bis 1 mm und besonders bevorzugt von 0,2 mm bis 0,5 mm bereitgestellt.

Es hat sich als besonders vorteilhaft herausgestellt, wenn die Maschenware als gestrickte Maschenware bereitgestellt wird. Weiter Möglichkeiten der Herstellung von Maschenware umfassen Häkeln oder Wirken.

Weitere Möglichkeiten bestehen in dem Bereitstellen des Drahtes und/oder der Faser als Gelege, Geflecht oder als Gewebe.

Bei der Herstellung der Maschenware hat sich die Herstellung mittels Rundstricken als besonders vorteilhaft erwiesen, da damit die Maschenware als Schlauch bereitgestellt wird und somit keine scharfen Kanten aufweist und nahtlos gestrickt bzw. rundgestrickt werden kann. Von einem solchen Endlosschlauch wird dann ein Stück der erforderlichen Länge abgeschnitten

Die Abmessungen des Schlauchs, d.h. sein Durchmesser und seine Länge hängen von den Abmessungen und den Eigenschaften, vor allem bezüglich Steifigkeit, Elastizität, Federung und Dämpfung, des zu bildenden Formkörpers ab und werden in einer Versuchsreihe empirisch bestimmt.

Es hat sich aus Gründen einer verbesserten Elastizität als außergewöhnlich vorteilhaft erwiesen, die Maschenware nicht unmittelbar nach dem Stricken zu verwenden, sondern anschließend noch einer formgebenden Behandlung zu unterwerfen. Bei dieser formgebenden Behandlung wird die Maschenware vorzugsweise verdichtet, wodurch sich der Draht und/oder die Faser enger aneinander legen.

In einem nächsten Verfahrensschritt wird die Maschenware oder der durch die Maschenware geformte Schlauch durch Pressen und/oder Walzen in die Form eine Bandes überführt.

Das Band wird dabei derart gepreßt und/oder gewalzt, so daß das Band bevorzugt eine Dicke von etwa 0,1 mm bis etwa 20 mm und besonders bevorzugt von etwa 0,75 mm bis etwa 2 mm aufweist. Die Maschenweite des Bandes dagegen entspricht dabei immer noch der Maschenweite des Schlauches .

In einem weiteren Verfahrensschritt wird das durch die Maschenware gebildete Band durch Verformen in eine wellige bzw. gewellte Form überführt wird. Das Band liegt nach diesem Verfahrensschritt somit im wesentlichen in Form einer Welle vor.

Das Band wird dabei derart in eine wellige Form überführt, daß die Wellenachsen parallel zueinander verlaufen.

Der Abstand zweier benachbarter Wellenberge weist einen Wert von 0,01 mm 100 mm, bevorzugt einen Wert von etwa 0,1 mm bis 5 mm und besonders bevorzugt einen Wert von etwa 1 mm bis etwa 3 mm auf. Der Höhenunterschied zwischen Wellental und Wellenberg weist einen Wert von etwa 0,01 mm bis etwa 100 mm und bevorzugt einen Wert von etwa 0,5 mm bis etwa 10 mm und besonders bevorzugt von 1 mm bis 5 mm auf.
Die wellige bzw. gewellte Form des Bandes wird dabei derart gewählt, so daß die Wellenachse, d.h. die Achse senkrecht zur Ausbreitungsrichtung der Welle, mit der Längsachse des Bandes einen Winkel α von etwa 0° bis etwa 90°, bevorzugt von etwa 10° bis etwa 60° und besonders bevorzugt von etwa 25° bis etwa 35° einschließt.

In einem nächsten Verfahrensschritt wird die Maschenware an sich oder die als gewelltes oder nicht gewelltes Band vorliegende Maschenware aufgerollt, gewickelt und/oder gefaltet.

Der so aufgerollte, gewickelte oder gefaltete Körper wird in einem nachfolgenden Verfahrensschritt einem formgebenden Prozeß unterworfen.

Durch einen Preßvorgang wird der aufgerollte, gewickelte oder gefaltete Körper in die Form eines gewünschten Formkörpers überführt.

In diesem formgebenden Prozeß mittels Pressen wird der durch das Band oder die Maschenware gebildete aufgerollte, gewickelte oder gefaltete Körper in seinem Volumen um einen Faktor von etwa 1/20 bis 9/10 bevorzugt von etwa 1/10 bis 1/2 und besonders bevorzugt von etwa 1/5 bis 1/3 reduziert. Da der Formkörper elastisch ist und sich nach dem Preßvorgang wieder dehnt, wird der Formkörper während der Kompression wesentlich stärker in seinen Abmessungen komprimiert. Die vorgenommene Kompression auf ein bestimmtes Volumen bestimmt zudem die Anzahl der zulässigen Belastungen und damit die Lebensdauer des erfindungsgemäßen Implantats.

Die Drähte und/oder Fasern der Maschenware verhaken und verklemmen sich durch den Preßvorgang untereinander und ineinander und verbinden sich darüber.

In diesem Verfahrensschritt bilden sich durch die Maschenware innerhalb des gepreßten Formkörpers Hohlräume aus, die, da das Ausgangsmaterial ein Gestrick war, zufällig aber gleichmäßig im Formkörper verteilt sind. Die innerhalb des gepreßten Formkörpers gebildeten Hohlräume weisen einen Durchmesser von etwa 0,001 mm bis etwa 10 mm, bevorzugt von etwa 0,04 mm bis etwa 2,0 mm und besonders bevorzugt von etwa 0,2 mm bis etwa 1,0 mm auf.

Durch das Herstellungsverfahren des Implantats kann gezielt Einfluß auf dessen Steifigkeit, Elastizität, Feder- und Dämpfungseigenschaften genommen werden. Die Eigenschaften werden bestimmt durch die gezielte Wahl des Materials, des Draht- bzw. Faserdurchmessers, der Maschenweite, der Höhe und der Breite der Wellen, sowie deren Winkel α zur Längsrichtung des Bandes und die Stärke der Kompression in dem Preßvorgang, da durch den gewählten Preßdruck die Verdichtung der Maschenware gezielt beeinflußt und somit die Größe der zwischen den Drähten und/oder Fasern verbliebenen Hohlräume bestimmt wird. Die einzelnen Parameter werden dabei in einer Versuchsreihe empirisch bestimmt. Somit können die Eigenschaften des zu formenden Formkörpers bzw. des zu formenden Implantats auf die Eigenschaften der zu ersetzenden Bandscheibe oder des zu ersetzenden Gelenks optimiert angepaßt werden, so daß das Implantat in seiner Funktion der örtlichen Beanspruchung optimal gerecht wird und eine verbesserte Kraftübertragung von dem Implantat in den Knochen ermöglicht.
Der durch den Preßvorgang gebildete Formkörper kann dabei im wesentlichen die gewünschte Form einer menschlichen Bandscheibe aufweisen. Der gebildete Formkörper ist nierenförmig mit einer gebogenen konvexen Seite und einer gegenüberliegenden konkaven Seite ausgebildet.

Beispielsweise kann eine nach diesem Verfahren gefertigte Bandscheibe mit den benachbarten Wirbeln mit auf dem Gebiet des Gelenkersatzes bekannten Verfahren verankert werden, beispielsweise über eine Presspassung oder durch ein Zementierverfahren.

Da der Formkörper durch die verdichtete Maschenware gebildet wird und somit neben den Hohlräumen eine rauhe Oberfläche aufweist, ermöglicht dies ein Einwachsen von Gewebe und den umgebenden Knochen fester mit dem Implantat zu verbinden. Dies erfordert keine weiteren Mittel, wie beispielsweise beim in der Beschreibung erwähnten Stand der Technik. Dort weisen die Implantate Vorsprünge auf, die die Scheibe gegenüber den Wirbeln positionieren und diese in der Stellung festhalten.

Gegenüber herkömmlichen Implantaten, die im allgemeinen mehrteilig ausgelegt sind und über ihre Komponenten in den Körper implantiert werden, kann das vorliegende Implantat als einteilige Einheit implantiert werden und somit eine übermäßige Belastung auf die umgebenden anatomischen Strukturen und somit den Patienten vermieden werden.

Die erfindungsgemäße Bandscheibe kann direkt als Implantat für den chirurgischen Einsatz bei Menschen oder Wirbeltieren zum Ersatz einer beschädigten Bandscheibe verwendet werden. Gegenstand der Erfindung ist aber auch die Verwendung des erfindungsgemäßen Implantats als Teil eines Bandscheibenimplantats, welches eine obere Platte, eine untere Platte und als elastischen Kern das genannte Implantat umfaßt, welches zwischen den Platten angeordnet ist und an diesen anliegt.
Daher kann die erfindungsgemäße Bandscheibe als Ersatz für den in der Beschreibung erwähnten elastomeren Kern in der DE 694 28 143 T2 eingesetzt werden und gewährt der dort beschriebenen Bandscheibe eine längere Lebenszeit. Weiter kann die verdichtete Maschenware auch als Kugelgelenklagersystem in der erwähnten DE 696 23 535 T2 eingesetzt werden und verleiht dem künstlichen Bandscheibenimplantat die Fähigkeit besitzt, Stöße auf die Wirbelsäule zu absorbieren.

In einer weiteren Ausführungsform kann der Formkörper zudem derart ausgebildet werden, so daß er im wesentlichen der Form einer natürlichen Hüftgelenkpfanne oder eines natürlichen Hüftgelenkkopfs entspricht. Weiterhin kann der Formkörper derart ausgebildet werden, so daß er im wesentlichen der Form einer natürlichen Schultergelenkpfanne oder eines natürlichen Schultergelenkkopfs entspricht. Ohne weitere Gelenke detailliert zu beschreiben, kann der erfindungsgemäße Formkörper auch derart ausgebildet werden, so daß er zum Ersatz, zum Teilersatz oder zum Verstärken für andere Gelenke im menschlichen Körper oder bei Wirbeltieren, beispielsweise Kniegelenke, Sprunggelenke, Ellenbogengelenke, Fingergelenke oder Zehengelenke, chirurgisch implantiert werden kann.

Das erfindungsgemäße Gelenk kann somit direkt als Implantat für den chirurgischen Einsatz bei Menschen oder Wirbeltieren zum Ersatz eines Gelenks verwendet werden. Gegenstand der Erfindung ist aber auch die Verwendung des erfindungsgemäßen Implantats als Teil eines Gelenkimplantats, umfassend eine Gelenkpfanne und/oder einen Gelenkkopf, welche jeweils zumindest durch Kunststoff, Metall oder Keramik gebildet werden und das genannte Implantat an der von den aneinander reibenden Gelenkflächen abgewandten Seite, d.h. in Richtung zum Knochen hin, angeordnet ist und an dieser anliegt. Die bekannten Werkstoffkombinationen zum Bilden einer Gelenkpaarung, d.h. zum Bilden von Gelenkpfanne und Gelenkkopf bzw. zum Bilden der aneinander reibenden Oberflächen von Gelenkpfanne und Gelenkkopf, wie beispielsweise Kunststoff-Kunststoff, Metall-Metall, Kunststoff-Metall, Kunststoff-Keramik oder Keramik-Keramik, sind in ihren Eigenschaften bezüglich Steifigkeit, Elastizität, Federung und Dämpfung nur mäßig anpaßbar, um den Funktionen des zu ersetzenden Gelenks gerecht zu werden.

Durch das Herstellungsverfahren des erfindungsgemäßen Implantats kann jedoch gezielt Einfluß auf dessen Steifigkeit, Elastizität, Feder- und Dämpfungseigenschaften genommen werden, so daß es an die Funktionen des zu ersetzenden Gelenks optimal angepaßt werden kann. Somit können hinsichtlich geringem Verschleiß durch verminderten Abrieb bewährte Gelenkpaarungen durch Kombination mit dem erfindungsgemäßen Implantat eine verbesserte, an die Knochenelastizität angepaßte, Elastizität aufweisen und somit eine optimierte Kraftübertragung von dem Implantat in den Knochen gewährleisten. Die Kraftübertragung von dem Implantat in den Knochen spielt dabei eine entscheidende Rolle bei der Frage der Langzeitverankerung des Implantats im Körper. Da zudem der Formkörper durch die verdichtete Maschenware gebildet wird und somit neben den Hohlräumen eine rauhe Oberfläche aufweist, ermöglicht dies zusätzlich ein Einwachsen von dem umgebenden Knochen, wodurch sich dieser fester mit dem Implantat verbindet, was eine Verbesserung der Langzeitverankerung des Implantats im Körper bedeutet.

In einer besonderen Ausführungsform kann in einem weiteren Verfahrensschritt das aus Maschenware gebildete Implantat mit Stammzellen besetzt werden, in welchem die Maschenware die Funktion eines Gerüstes übernimmt.

Zudem umfaßt die vorliegende Erfindung ein Implantat für den chirurgischen Einsatz bei Menschen oder Wirbeltieren zum Ersatz, zum Teilersatz oder zum Verstärken einer beschädigten Bandscheibe oder zum Ersatz, zum Teilersatz oder zum Verstärken eines Gelenks, welches mit dem vorstehenden Verfahren herstellbar ist.

Gegenstand der Erfindung ist zudem die Verwendung von Maschenwaren zur Herstellung von Implantaten für den chirurgischen Einsatz bei Menschen oder Wirbeltieren zum Ersatz, zum Teilersatz oder zur Verstärkung einer beschädigten Bandscheibe oder zum Ersatz, zum Teilersatz oder zur Verstärkung eines Gelenks.

Weiter umfasst die Erfindung ein Implantat, welches einen vom Körper resorbierbaren Draht und/oder eine resorbierbare Faser umfasst und der Draht oder die Faser zumindest abschnittsweise mit Stammzellen besetzt ist.

Dieses erfindungsgemäße Implantat eignet sich als Ersatzmaterial für verschiedenste Anwendungen. Beispielsweise könnten bei Knochenbrüchen, deren Heilung sich als schwierig erweist, mit Stammzellen besetzte Gestricke verwendet werden, mit deren Hilfe der Knochen eingeschlossen und stabilisiert werden könnte. Durch die Stammzellen kann sich beispielsweise neues Knochenmaterial bilden.

Denkbar ist, unterschiedlichste Teile eines Körpers mit einem mit Stammzellen besetzen Gestrick zu ersetzen.
Die Erfindung soll im Folgenden anhand eines Ausführungsbeipieles erläutert werden.

Fig. 1 zeigt eine schematische Darstellung einer erfindungsgemäßen Bandscheibenprothese.

Fig. 2 zeigt die schematische Darstellung eines Gestricks, aus dem die Bandscheibenprothese gemäß der Fig. 1 geformt wird.

Fig. 3 zeigt eine Detailansicht von Fig. 2.

Fig. 4 zeigt eine beispielhafte Kraft-Weg-Kurve einer erfindungsgemäßen Bandscheibenprothese.

Fig. 5 zeigt ebenfalls beispielhafte Kraft-Weg-Kurven von erfindungsgemäßen Bandscheibenprothesen.

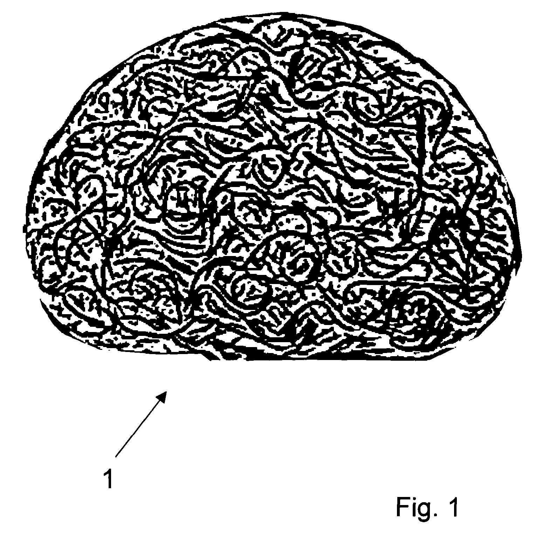

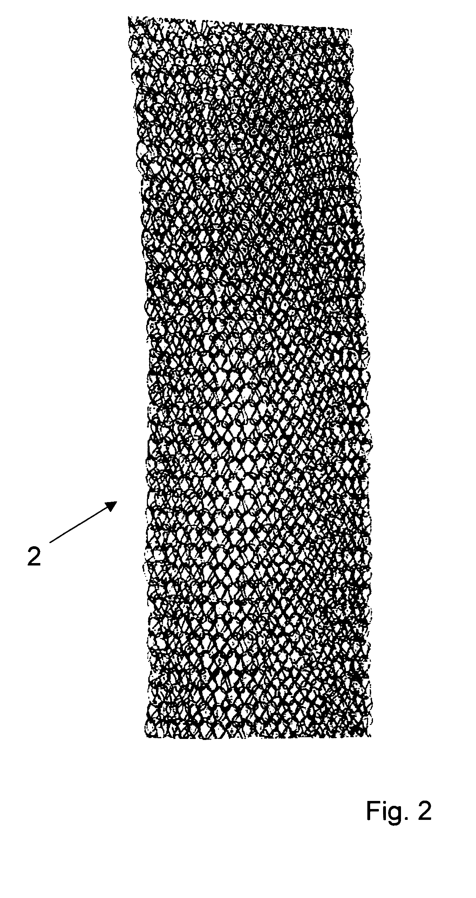

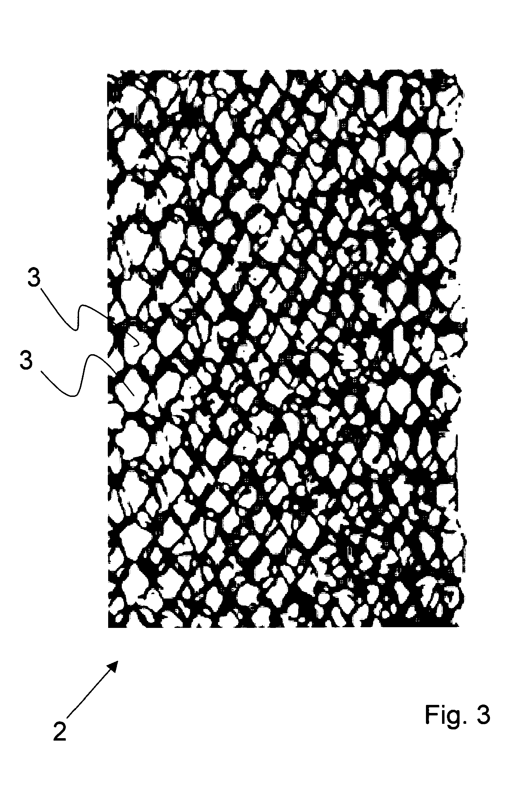

Fig. 1 zeigt eine Bandscheibenprothese 1 in der Draufsicht. Die Bandscheibenprothese 1 besitzt etwa den Querschnitt einer natürlichen Bandscheibe. Die Bandscheibenprothese 1 gemäß dem Ausführungsbeispiel besteht aus Titandraht mit einem Durchmesser von 0,23 mm, ist etwa 5 mm hoch und wiegt ca. 4 g. Der Titandraht wurde zunächst als Maschenware verstrickt. Das Gestrick (Fig. 2 und 3) wurde in die Form der Bandscheibenprothese 1 gepresst. Die so entstandene Bandscheibenprothese 1 ist biokompatibel und passt sich durch ihre elastische Struktur den Wirbelkörpern an. Die Oberfläche der Bandscheibenprothese 1 geht mit dem biologischen Gewebe eine teils formschlüssige Verbindung ein, so daß die Bandscheibenprothese 1 ohne weitere Mittel gegen Herausrutschen gesichert ist.

Fig. 2 zeigt das Ausgangsmaterial für ein erfindungsgemäßes Implantat. Es handelt sich dabei um ein Gestrick 2 aus Titandraht, welches die Form eines Schlauchs aufweist. Das Gestrick wird in die Form eines Implantats gepresst.

Fig. 3 zeigt eine Detailansicht der Fig. 2. Gut zu erkennen ist, wie die einzelnen Maschen 3 ineinander eingreifen.

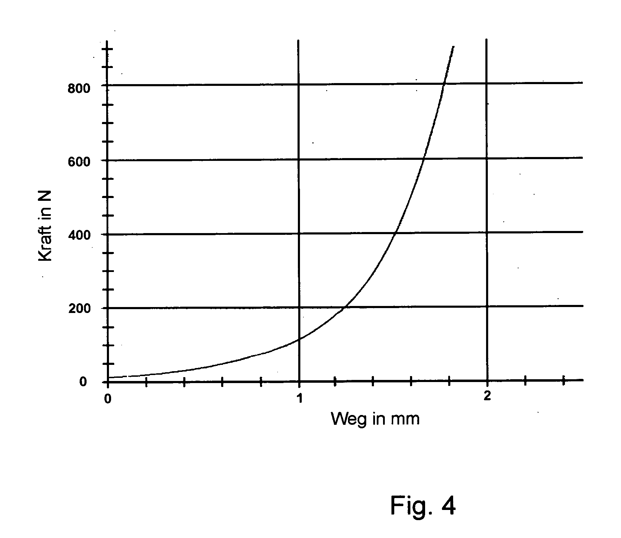

Fig. 4 zeigt einen beispielhaften Kraft-Weg-Verlauf einer erfindungsgemäßen Bandscheibenprothese. Auf der x-Achse ist die Verformung der Bandscheibe in mm aufgetragen, auf der y-Achse die Kraft in N. Zu erkennen ist, daß kein linearer Kraft-Weg-Verlauf vorliegt, sondern daß die Kraft in etwa quadratisch bis kubisch ansteigt. Die Prothese federt also zunächst weich und wird dann härter. Die Federkonstante F der Bandscheibenprothese steigt dabei in etwa linear bis quadratisch an.

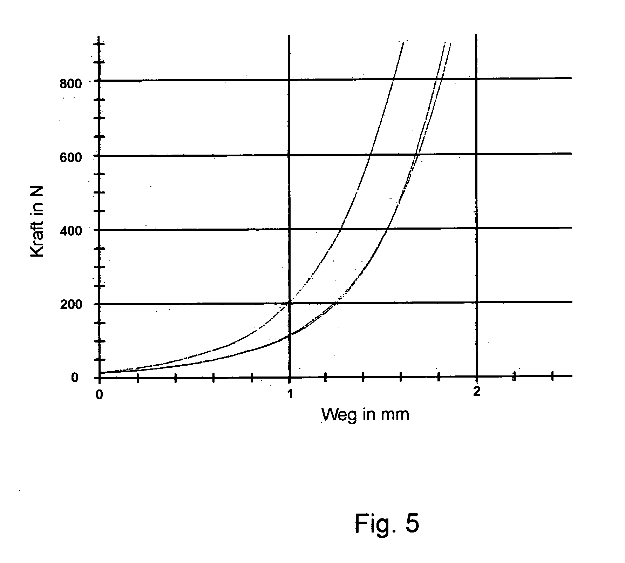

Fig. 5 zeigen weitere Kurven eines beispielhaften Kraft-Weg-Verlaufs verschiedener Ausführungsbeispiele einer Bandscheibenprothese. Die Charakteristik der Prothese kann variiert werden.

## Patentansprüche

1. Implantat für den chirurgischen Einsatz bei Menschen oder Wirbeltieren zum Ersatz, zum Teilersatz oder zum Verstärken einer beschädigten Bandscheibe oder zum Ersatz, zum Teilersatz oder zum Verstärken eines Gelenks, welches zumindest einen Draht und/oder zumindest eine Faser umfaßt,
**dadurch gekennzeichnet, daß**
der Draht und/oder die Faser zumindest teilweise als gepreßte Maschenware vorliegt.

2. Implantat nach Anspruch 1 **dadurch gekennzeichnet, daß** der Draht und/oder die Faser Teil eines Gestricks ist.

3. Implantat nach Anspruch 1 oder 2 **dadurch gekennzeichnet, daß**
der Draht und/oder die Faser zumindest ein biokompatibles Material, insbesondere Titan, Edelstahl, Edelstahllegierungen oder Titanlegierungen umfaßt bzw. umfassen.

4. Implantat nach einem der vorstehenden Ansprüche **dadurch gekennzeichnet, daß**
der Draht und/oder die Faser einen Durchmesser von etwa 0,01 mm bis 5 mm, bevorzugt von etwa 0,05 mm bis etwa 1 mm und besonders bevorzugt von etwa 0,2 mm bis etwa 0,3 mm aufweist bzw. aufweisen.

5. Implantat nach einem der vorstehenden Ansprüche **dadurch gekennzeichnet, daß**
die Maschenware eine Maschenweite von 0,01 mm bis 50 mm, bevorzugt von 0,5 mm bis 20 mm und besonders bevorzugt von 3 mm bis 8 mm aufweist.

6. Implantat nach einem der vorstehenden Ansprüche **dadurch gekennzeichnet, daß**
die Maschenware als Schlauch vorliegt.

7. Implantat nach einem der vorstehenden Ansprüche **dadurch gekennzeichnet, daß**
die Maschenware als gepreßter Formkörper vorliegt.

8. Implantat nach Anspruch 7, **dadurch gekennzeichnet, daß** der Formkörper eine Federkonstante aufweist, die bei zunehmender elastischer Verformung ansteigt.

9. Implantat nach Anspruch 8, **dadurch gekennzeichnet, daß** die Federkonstante zwischen 5 und 3000 N/mm beträgt.

10. Verfahren zur Herstellung eines Implantats für den chirurgischen Einsatz bei Menschen oder Wirbeltieren, insbesondere zur Herstellung eines Implantats gemäß den vorigen Ansprüchen, zum Ersatz, zum Teilersatz oder zum Verstärken einer beschädigten Bandscheibe oder zum Ersatz, zum Teilersatz oder zum Verstärken eines Gelenks, welches das Bereitstellen zumindest eines Drahtes und/oder zumindest einer Faser umfaßt,
**dadurch gekennzeichnet, dass** der Draht und/oder die Faser als Teil einer Maschenware bereit gestellt wird und die Maschenware gepresst wird.

11. Verfahren nach Anspruch 10 **dadurch gekennzeichnet, daß** der Draht und/oder die Fasern zumindest teilweise als Maschenware bereitgestellt wird bzw. werden.

12. Verfahren nach einem der vorstehenden Ansprüche **dadurch gekennzeichnet, daß**
die Maschenware als Schlauch bereitgestellt wird.

13. Verfahren nach dem Anspruch 12 **dadurch gekennzeichnet, daß**
die Maschenware oder der durch die Maschenware geformte Schlauch durch Pressen und/oder Walzen in die Form eine Bandes überführt wird.

14. Verfahren nach einem der vorstehenden Ansprüche 10 bis 11 **dadurch gekennzeichnet, daß**
die Maschenware aufgerollt, gewickelt und/oder gefaltet wird.

15. Verfahren nach einem der vorstehenden Ansprüche 10 bis 11 **dadurch gekennzeichnet, daß**
in einem formgebenden Prozeß mittels Pressen die Maschenware in die Form eines gewünschten Formkörpers ausgebildet wird.
